# EUROPEAN PATENT SPECIFICATION

(11) **EP 1 465 553 B1**
(45) Date of publication and mention of the grant of the patent: **13.06.2007**
(21) Application number: 03713201.6
(22) Date of filing: 03.01.2003
(51) Int. Cl.: A61F 2/82

(54) **IMPROVED EXTERIOR STENT**
VERBESSERTER EXTERNER STENT
ENDOPROTHESE VASCULAIRE A EXTERIEUR AMELIORE

(30) Priority: 03.01.2002 US 37817; 02.01.2003 US 335680
(43) Date of publication of application: 13.10.2004
(73) Proprietor: BRAGINSKY, Sidney, Dix Hills, NY 11746 (US); Houser, Russell A., Livermore, CA 94550 (US)
(72) Inventor: BRAGINSKY, Sidney, Dix Hills, NY 11746 (US); Houser, Russell A., Livermore, CA 94550 (US)
(74) Representative: Müller-Boré & Partner Patentanwälte
(86) International application number: PCT/US2003/000188
(87) International publication number: WO 2003/057080

(56) References cited:
- GB-A- 2 344 053
- US-A- 5 603 720

## Description

### BACKGROUND OF THE INVENTION

Intralumenal grafting and stents are used to hold a body lumen open and enlarged. This involves the percutaneous insertion and placement by catheter of a cylindrical prosthetic device within a body lumen. Stents are used in the vascular system, respiratory, biliary and urinary tracts. Typically, they are composed of stainless steal springs, wire in a zigzag pattern or helically wound springs.

Intralumenal grafts create several potentially dangerous conditions. If the grafts are under expanded at their target location in the lumen or under sized for the lumen, they do not secure themselves properly and can migrate away from the location. An over expanded or oversized graft can rupture the lumen.

In GB 2 344 053 A a stent for internally or externally supporting part of a blood vessel is disclosed, where the stent is adapted to flex three-dimensionally. By maintaining a non-planar curvature in the vessel itself, favourable blood flow velocity patterns can be achieved through generation therein of 'swirl' flow, which reduce failures in such vessels through diseases such as thrombosis, atherosclerosis, and the like.

### SUMMARY OF THE INVENTION

The present invention is directed to a prosthesis that is capable of opening and enlarging body lumens from the exterior. The invention provides for external, a traumatic vessel support. The support, which can be interchangeably referred to as a stent, may be made from a biologically inert material and may be reinforced with a layer consisting of a polymer or metallic braid, wrap or other pattern. It may be secured to the exterior of the lumen using an adhesive, protruding member, suture or a combination of these means.

The result of the use of the present invention is a larger and reinforced lumenal vessel diameter. One effect of increased lumenal diameter is an increase in blood flow for cardiovascular vessels.

It is an object of the present invention to provide an a traumatic exterior lumenal stent.

It is another object of the present invention to provide an a traumatic exterior lumenal stent that enlarges lumenal diameters.

It is still another object of the present invention to provide a device to increase lumenal diameter without implanting a foreign object into the body lumen, thereby reducing the risk of lumenal rupture and incidences of acute thrombosis and intimal hyperplasia.

### BRIEF DESCRIPTION OF THE DRAWINGS

Figure 1 is a schematic depiction of the a two-piece stent with a hinge.
Figure 2 is a depiction of a bifurcated stent without seam detail.
Figure 3 is a depiction of the cross-section of an embodiment of a one-piece stent.
Figure 4 is a schematic depiction of a body lumen that is a candidate for the presently claimed process and device.
Figures 5 and 6 depict an embodiment of the claimed process of providing exterior lumenal in a lumen having a lesion.
Figure 7 depicts a lumen having had the presently claimed device applied and the presently claimed method performed thereon.

### DETAILED DESCRIPTION OF THE INVENTION

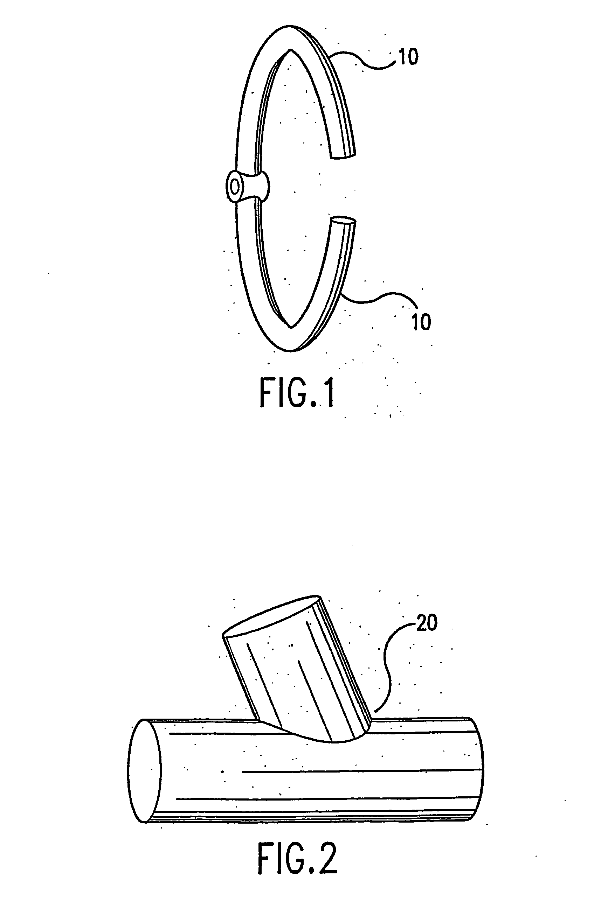

This stent may comprise a single or multiple pieces. In Fig. 1 the multi-piece piece design would be constructed as two semi-cylindrical elements that are integrally connected at a hinge or flexible element. The two semi-cylindrical elements 10 are constructed to connect to each other opposite the hinge. The ends opposite of the hinge or flexible element are fastened together by a latch or other conventional means that will hold the device in a cylindrical shape.

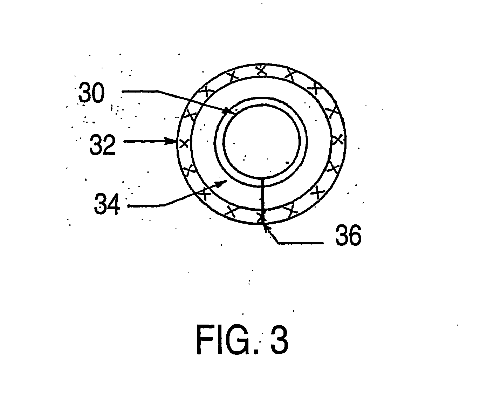

Figure 3 shows a cross-section of an embodiment of the invention, wherein the stent is a single piece. A seam 36 runs the width of the cross-sectional area. It will be appreciated that the multi-piece design can posses the same cross-sectional features as said multi-piece design may be comprised of the same layered structure.

In an embodiment, the stent is has multiple layers and an outer layer 32 for reinforcement. In this embodiment, a support layer is an interior layer 34 and may be made from a soft, compliant, a traumatic material. For example, the interior layer may be made from ePTFE, woven Dacron, or other compliant material. Alternate embodiments may or may not contain the outer reinforcement layer. In said alternate embodiments, the support layer 34, which in this embodiment is equivalent to the interior layer of the embodiment having the outer reinforcement layer, may be made from expanded PTFE (ePtfe), woven Dacron or other suitable material. Alternatively, it may be made from biodegradable material, including but not limited to collagen, synthetic polymers such as polyglycolic acids, polylactids, polyhydroxybutyrates, polyhydroxyvaleriates, polydioxanons, modified starches, gelatins, modified celluloses, polyglycols, polyacrylic acids, polymethacrylic acids, natural or synthetic aliphatic or hydroxy polymer of lactic acid, glycolic acid or some combination of these or others. In an embodiment, the outer layer 32 is provided and is stronger and less compliant than the inner layer. In this embodiment, the outer layer is comprised of a polymer or metallic braid, wrap or other pattern. The whole stent may also be flexible.

On the inner diameter of the support layer is a securing element 30. In an embodiment, an inner diameter of the interior layer may be coated or comprised of a biologically inert adhesive, which acts as the securing element. The adhesive is used to adhere to the exterior of the target body lumen. The stent may be secured to the lumen wall by a securing element such as an adhesive 30 such as cyanoacrylate, fibrin, fibrinogen, or some combination of these. However the adhesive is not limited to these materials. The adhesive could have a surface contact cure method such as UV light, heat or some combination. Alternatively, the stent may be secured by alternative securing means such as penetrating barbs or hooks, or reversible adhesive bonding between the lumen and stent. Additionally, conventional locking tabs or a ratchet system may be used to tighten and secure the stent onto the lumen.

The device is constructed in different sizes to be able to approximate the size of the target lumen or is able to expand or contract to properly reach the lumen size.

Extrusion, molding, dip coating or a combination of these methods or other methods may be used to manufacture the stent. The stent may be made from a single piece and may have a porous or corrugated surface on one or both the exterior and interior sides. The stent may have holes or groves completely through it to provide the vessel with nutrients.

During fabrication, the stent can be annealed around an appropriately sized rod or mandrel, to increase radial strength as well as dimensional and geometric stability.

One or more layers of the stent may be braided, woven, wrapped or other pattern. The stent may have-layers consisting of polymer, metal or a combination for surface texture and strength.

A method of use for the stent includes placing it around the exterior of the lumen and closing it into its cylindrical shape. The lumen is pushed against the interior of the stent where it comes into contact with the adhesive and sticks to the interior of the stent thereby enlarging and opening the lumen. The lumen is expanded to push against the stent by an angioplasty balloon or other similar device. It should be noted that the stent need not provide complete circumferential contact. The design could offer sufficient reinforcement to the vessel with as little as 40% coverage and possibly less.

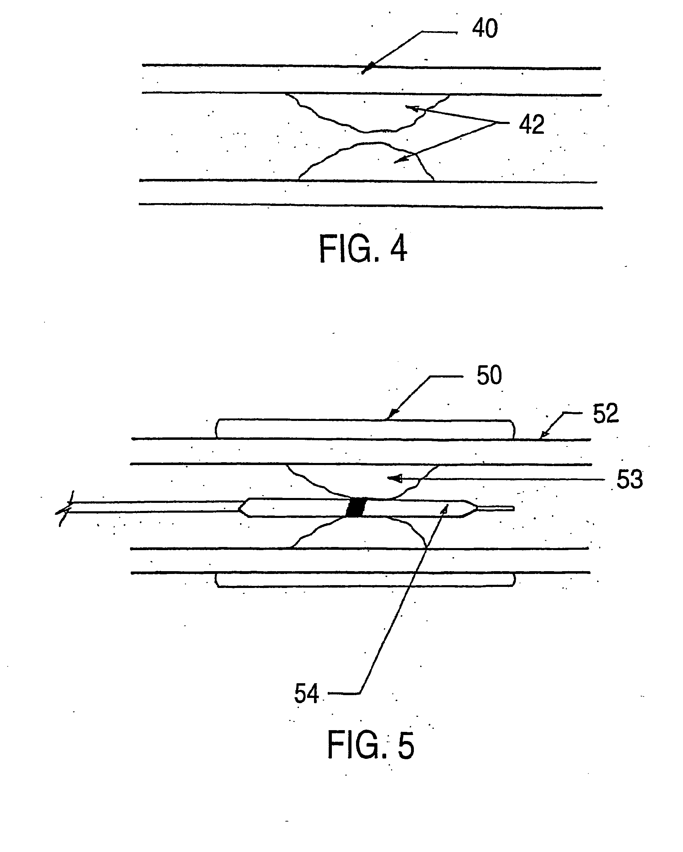

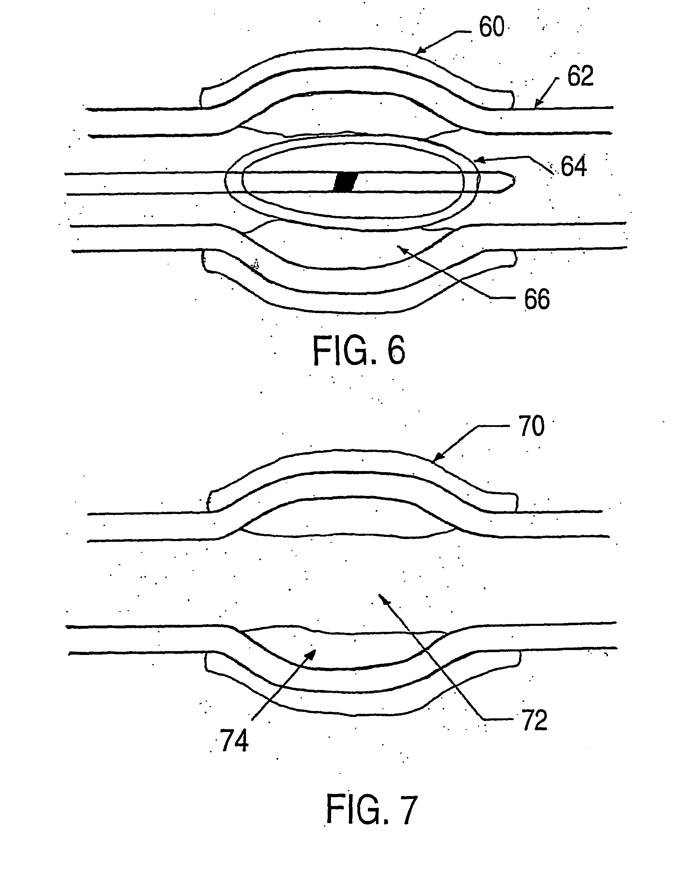

The stent may be placed around a target lumen such as the one in Figure 4, having a lesion 42, and ratcheted or compressed down to contact the exterior of the lumen wall. The contact is sufficient when the lumen becomes secure to the stent by the barbs or adhesive or other securing means. Alternatively, a PTCA balloon catheter depicted as 54 in Figure 5 may be advanced within the lumen 52 into place with the balloon positioned at the site of the external stent 50. As shown in Figure 6, the balloon 64 is contact with a lesion 66 (also depicted as 53 in Figure 5) then inflated causing the lumen to expand and come into contact with the interior side of the stent bonding the lumen to the stent. After the stent 70 is secured, the balloon is then deflated and the catheter withdrawn, resulting in the lumen having a larger diameter 72 as the narrowing caused by the lesion 74 in Figure 7 has been widened.

In an alternative embodiment and as shown in Fig. 2, the stent may be bifurcated or more to provide for coverage of bifurcated lumens.

Accordingly, it should be readily appreciated that the exterior stent and the uses of the present invention has many practical applications. Additionally, although the preferred embodiment has been illustrated and described, it will be obvious to those skilled in the art that various modifications can be made without departing from the scope of this invention.

## Claims

1. An exterior stent (50, 60, 70) comprising:
a support layer (34) having an inner diameter and an outer diameter, and
a securing element (30)
**characterized in that**
the securing element (30) is arranged on the inner diameter of the support layer (34) wherein said securing element (30) is contactable with an exterior diameter of a body lumen (40, 52, 62).

2. A stent (50, 60, 70) as in claim 1, wherein said securing element (30) is contactable with an exterior diameter of a body lumen (40, 52, 62) having a smaller diameter than the inner diameter of the support layer (34).

3. A stent (50, 60, 70) as in claim 1 wherein: the support layer (34) comprises biologically inert material.

4. A stent (50, 60, 70) as in claim 3 wherein: the inert material is a shape-memory material or PTFE or Dacron.

5. A stent (50, 60, 70) as in claim 3 wherein: the support layer (34) further comprises a biologically active material, the active material being a drug-releasing coating on a surface of the stent (50, 60, 70) that permits timed or prolonged pharmacological activity.

6. A stent (50, 60, 70) as in claim 1 wherein: the support layer (34) comprises resorbable material.

7. A stent (50, 60, 70) as in claim 1 wherein: the support layer (34) is shape-memory material.

8. A stent (50, 60, 70) as in claim 1 wherein: the support layer (34) is porous for providing nutrients or irrigation to the lumen (40, 52, 62).

9. A stent (50, 60, 70) as in claim 1 wherein: the support layer (34) comprises a braided material.

10. A stent (50, 60, 70) as in claim 1 wherein: the support layer (34) is a single unified member.

11. A stent (50, 60, 70) as in claim 1 wherein: the support layer (34) comprises at least two members flexibly joined together.

12. A stent (50, 60, 70) as in claim 11 wherein: the members are joined by a hinge,

13. A stent (50, 60, 70) as in claim 1 wherein: the support layer (34) is bifurcated.

14. A stent (50, 60, 70) as in claim 1 wherein: the support layer (34) comprises a radioactive element for delivering radiation directly to the lumen (40, 52, 62).

15. A stent (50, 60, 70) as in one of the claims 3, 6, and14 wherein: the support layer (34) further comprises a biologically active material.

16. A stent (50, 60, 70) as in claim 1 wherein: the securing element (30) is a barb or a hook or a suture.

17. A stent (50, 60, 70) as in claim 1 wherein: the securing element (30) is an adhesive.

18. A stent (50, 60, 70) as in claim 17 wherein: the adhesive is biologically inert.

19. A stent (50, 60, 70) as in claim 17 wherein: the adhesive requires curing.

20. A stent (50, 60, 70) as in claim 1 wherein: the securing element (30) are locks suited to close the stent tightly onto the lumen (40, 52, 62) to prevent it from slipping but not to restrict the lumen (40, 52, 62).

21. A stent (50, 60, 70) as in claim 1 wherein: the support layer (34) is suited to cover less than the entire circumference of the lumen (40, 52, 62).

22. A stent (50, 60, 70) as in claim 1 further comprising: a reinforcing layer for strengthening the support layer (34), the reinforcing layer preferably comprising a braided material.

## Patentansprüche

1. Externer Stent (50, 60, 70), umfassend:
eine Trage- bzw. Stützschicht (34) mit einem Innendurchmesser und einem Außendurchmesser, und
ein Sicherungselement (30),
**dadurch gekennzeichnet, dass**
das Sicherungselement (30) am Innendurchmesser der Stützschicht (34) angeordnet ist, wobei das Sicherungselement (30) mit einem Außendurchmesser eines Körperlumens (40, 52, 62) in Kontakt gebracht werden kann bzw. kontaktierbar ist.

2. Stent (50, 60, 70) nach Anspruch 1, wobei das Sicherungselement (30) mit einem Außendurchmesser eines Körperlumens (40, 52, 62), das einen kleineren Durchmesser als der Innendurchmesser der Stützschicht (34) aufweist, in Kontakt gebracht werden kann.

3. Stent (50, 60, 70) nach Anspruch 1, wobei die Stützschicht (34) ein biologisch inertes Material umfasst.

4. Stent (50, 60, 70) nach Anspruch 3, wobei das inerte Material ein Formgedächtnismaterial oder PTFE oder Dacron ist.

5. Stent (50, 60, 70) nach Anspruch 3, wobei die Stützschicht (34) ferner ein biologisch aktives Material umfasst, wobei das aktive Material eine Arzneimittel freisetzende Beschichtung auf einer Oberfläche bzw. Fläche des Stents (50, 60, 70) ist, die eine zeitgesteuerte oder verlängerte pharmakologische Aktivität zulässt.

6. Stent (50, 60, 70) nach Anspruch 1, wobei die Stützschicht (34) resorbierbares Material umfasst.

7. Stent (50, 60, 70) nach Anspruch 1, wobei die Stützschicht (34) ein Formgedächtnismaterial ist.

8. Stent (50, 60, 70) nach Anspruch 1, wobei die Stützschicht (34) porös ist, um dem Lumen (40, 52, 62) Nährstoffe oder Irrigation zuzuführen.

9. Stent (50, 60, 70) nach Anspruch 1, wobei die Stützschicht (34) ein geflochtenes Material umfasst.

10. Stent (50, 60, 70) nach Anspruch 1, wobei die Stützschicht (34) eine einzelne einheitliche Komponente bzw. ein einzelnes einheitliches Glied ist.

11. Stent (50, 60, 70) nach Anspruch 1, wobei die Stützschicht (34) mindestens zwei flexibel miteinander verbundene Komponenten bzw. Glieder umfasst.

12. Stent (50, 60, 70) nach Anspruch 11, wobei die Komponenten durch ein Gelenk verbunden sind.

13. Stent (50, 60, 70) nach Anspruch 1, wobei die Stützschicht (34) gegabelt ist.

14. Stent (50, 60, 70) nach Anspruch 1, wobei die Stützschicht (34) ein radioaktives Element umfasst, um dem Lumen (40, 52, 62) direkt Strahlung zuzuführen.

15. Stent (50, 60, 70) nach einem der Ansprüche 3, 6 und 14, wobei die Stützschicht (34) ferner ein biologisch aktives Material umfasst.

16. Stent (50, 60, 70) nach Anspruch 1, wobei das Sicherungselement (30) ein Widerhaken oder ein Haken oder eine Naht ist.

17. Stent (50, 60, 70) nach Anspruch 1, wobei das Sicherungselement (30) ein Klebstoff ist.

18. Stent (50, 60, 70) nach Anspruch 17, wobei der Klebstoff biologisch inert ist.

19. Stent (50, 60, 70) nach Anspruch 17, wobei der Klebstoff ein Aushärten erfordert.

20. Stent (50, 60, 70) nach Anspruch 1, wobei das Sicherungselement (30) Verriegelungen sind, die geeignet sind, den Stent eng um bzw. auf das Lumen (40, 52, 62) zu schließen, um zu verhindern, dass es rutscht, ohne jedoch dabei das Lumen (40, 52, 62) zu behindern.

21. Stent (50, 60, 70) nach Anspruch 1, wobei die Stützschicht (34) geeignet ist, weniger als den gesamten Umfang des Lumens (40, 52, 62) zu bedecken.

22. Stent (50, 60, 70) nach Anspruch 1, der ferner eine Verstärkungsschicht zum Verstärken der Stützschicht (34) umfasst, wobei die Verstärkungsschicht bevorzugt ein geflochtenes Material umfasst.

## Revendications

1. Endoprothèse vasculaire à extérieur amélioré (50, 60, 70) comprenant:
une couche de support (34) ayant un diamètre interne et un diamètre externe, et un élément de fixation (30),
**caractérisée en ce que**
l'élément de fixation (30) est disposé sur le diamètre interne de la couche de support (34), dans laquelle ledit élément de fixation (30) peut être mis en contact avec un diamètre extérieur d'une lumière de corps (40, 52, 62).

2. Endoprothèse vasculaire (50, 60, 70) selon la revendication 1, dans laquelle ledit élément de fixation (30) peut être mis en contact avec un diamètre extérieur d'une lumière de corps (40, 52, 62) ayant un diamètre inférieur au diamètre interne de la couche de support (34).

3. Endoprothèse vasculaire (50, 60, 70) selon la revendication 1, dans laquelle: la couche de support (34) comprend un matériau biologiquement inerte.

4. Endoprothèse vasculaire (50, 60, 70) selon la revendication 3, dans laquelle le matériau inerte est un matériau à mémoire de forme ou PTFE ou Dacron.

5. Endoprothèse vasculaire (50, 60, 70) selon la revendication 3, dans laquelle : la couche de support (34) comprend en outre un matériau biologiquement actif, le matériau actif étant un revêtement à libération de médicament sur une surface de l'endoprothèse vasculaire (50, 60, 70) qui permet une activité pharmacologique temporisée ou prolongée.

6. Endoprothèse vasculaire (50, 60, 70) selon la revendication 1, dans laquelle la couche de support (34) comprend un matériau résorbable.

7. Endoprothèse vasculaire (50, 60, 70) selon la revendication 1, dans laquelle la couche de support (34) est un matériau à mémoire de forme.

8. Endoprothèse vasculaire (50, 60, 70) selon la revendication 1, dans laquelle la couche de support (34) est poreuse pour fournir des nutriments ou une irrigation à la lumière (40, 52, 62).

9. Endoprothèse vasculaire (50, 60, 70) selon la revendication 1, dans laquelle la couche de support (34) comprend un matériau tressé.

10. Endoprothèse vasculaire (50, 60, 70) selon la revendication 1, dans laquelle la couche de support (34) est un élément unifié simple.

11. Endoprothèse vasculaire (50, 60, 70) selon la revendication 1, dans laquelle : la couche de support (34) comprend au moins deux éléments reliés ensemble de manière flexible.

12. Endoprothèse vasculaire (50, 60, 70) selon la revendication 11, dans laquelle les éléments sont reliés par une articulation,

13. Endoprothèse vasculaire (50, 60, 70) selon la revendication 1, dans laquelle la couche de support (34) est jumelé.

14. Endoprothèse vasculaire (50, 60, 70) selon la revendication 1, dans laquelle la couche de support (34) comprend un élément radioactif pour fournir une radiation directement à la lumière (40, 52, 62).

15. Endoprothèse vasculaire (50, 60, 70) selon l'une des revendications 3, 6 et 14, dans laquelle : la couche de support (34) comprend en outre un matériau biologiquement actif.

16. Endoprothèse vasculaire (50, 60, 70) selon la revendication 1, dans laquelle l'élément de fixation (30) est un aiguillon ou un crochet ou une suture.

17. Endoprothèse vasculaire (50, 60, 70) selon la revendication 1, dans laquelle l'élément de fixation (30) est un adhésif.

18. Endoprothèse vasculaire (50, 60, 70) selon la revendication 17, dans laquelle l'adhésif est biologiquement inerte.

19. Endoprothèse vasculaire (50, 60, 70) selon la revendication 17, dans laquelle l'adhésif exige une réticulation.

20. Endoprothèse vasculaire (50, 60, 70) selon la revendication 1, dans laquelle l'élément de fixation (30) est représenté par des verrous adaptés pour fermer l'endoprothèse vasculaire étroitement sur la lumière (40, 52, 62) afin d'éviter qu'elle ne glisse mais pas pour limiter la lumière (40, 52, 62).

21. Endoprothèse vasculaire (50, 60, 70) selon la revendication 1, dans laquelle : la couche de support (34) est adaptée pour couvrir moins que la circonférence totale de la lumière (40, 52, 62).

22. Endoprothèse vasculaire (50, 60, 70) selon la revendication 1, comprenant en outre : une couche de renfort pour renforcer la couche de support (34), la couche de renfort comprenant de préférence un matériau tressé.
